(19) Europäisches Patentamt
European Patent Office
Office européen des brevets

(11) Publication number: 0 430 518 A2

# (12) EUROPEAN PATENT APPLICATION

(21) Application number: **90312548.2**

(51) Int. Cl.[5]: **C07D 473/00**, A61K 31/52

(22) Date of filing: **19.11.90**

(30) Priority: **24.11.89 GB 8926623**

(43) Date of publication of application:
**05.06.91 Bulletin 91/23**

(84) Designated Contracting States:
**AT BE CH DE DK ES FR GB GR IT LI LU NL SE Bulletin**

(71) Applicant: **GLAXO GROUP LIMITED**
**Clarges House 6-12 Clarges Street**
**London W1Y 8DH(GB)**

(72) Inventor: **Storer, Richard, Glaxo Group Res. Ltd.**
**Berkley Avenue**
**Greenford, Middlesex UB6 0HE(GB)**
Inventor: **Paternoster, Ian Leonard, Glaxo Group Res. Ltd.**
**Berkley Avenue**
**Greenford, Middlesex UB6 0HE(GB)**
Inventor: **Borthwick, Alan David, Glaxo Group Res. Ltd.**
**Berkley Avenue**
**Greenford, Middlesex UB6 0HE(GB)**
Inventor: **Biggadike, Keith, Glaxo Group Res. Ltd.**
**Berkley Avenue**
**Greenford, Middlesex UB6 0HE(GB)**

(74) Representative: **Hildyard, Edward Martin et al**
**Frank B. Dehn & Co. European Patent Attorneys Imperial House 15-19 Kingsway**
**London WC2B 6UZ(GB)**

## (54) Cyclopentane derivatives.

(57) (1'S,3'S,4'S)-2-amino-1,9-dihydro-9-(3,4-dihydroxy-3- hydroxymethyl-1-cyclopentyl)-6H-purin-6-one, sodium salt and pharmaceutical formulations thereof and processes for its preparation are described. The compound may be used in the treatment of viral infections, particularly those caused by Herpesviridae.

EP 0 430 518 A2

# CYCLOPENTANE DERIVATIVE

This invention relates to a new antiviral compound, to processes for its preparation, to pharmaceutical compositions containing it and to its use in medicine.

Existing treatments for viral infections include the administration of chemical compounds which are nucleoside analogues, for example 2′-deoxy-5-iodouridine, 9-(2-hydroxyethoxymethyl)guanine and 9-β-D-arabinofuranosyladenine. Carbocyclic analogues of nucleosides are also known to have effect against certain viruses, and in GB-A-21294245, GB-A-2179349 and J. Med. Chem. 1984, 27, 1416-21 carbocyclic analogues of nucleosides are disclosed having activity against strains of herpes simplex virus types I and II. There is however a need for compounds with good antiviral activity coupled with lower levels of cytotoxicity.

We have now found a new compound which has shown good activity against viruses, especially Herpesviridae, whilst having a low level of cytotoxicity. Thus, according to one aspect, the present invention provides (1′,3′S,4′S)-2-amino- 1,9-dihydro-9-(3,4-dihydroxy-3- hydroxymethyl-1-cyclopentyl)-6H-purin-6-one, sodium salt (referred to below as Compound A).

The parent of Compound A [i.e. the compound (1′S,3′S,4′S)-2- amino-1,9-dihydro-9-(3,4-dihydroxy-3-hydroxymethyl-1-cyclopentyl)-6H- purin-6-one)] is described in our European Patent Application Publication No. 0345076, together with other antiviral carbocyclic analogues of nucleosides and their salts and solvates. Compound A is advantageous as compared to these compounds generally and in particular to the parent of Compound A as regards its use in medicine. Thus, for example, Compound A has improved solubility in water which is a desirable characteristic for pharmaceutical formulation, especially in the preparation of formulations for parenteral (e.g. intravenous) administration.

We have found that Compound A is highly potent in vitro and in vivo against strains of both herpes simplex virus types I and II whilst having a low level of cytotoxicity. In vitro testing was carried out using the standard plaque reduction test whilst in vivo testing was carried out on the mouse according to the method described by Ericson et al. (1985) Antimicrobial Agents - Chemotherapy 27, 753-759.

It should be noted that Compound A lacks a glycosidic bond which forms a site for both chemical and biological cleavage. Stability against glycosidic cleavage is, of course, a valuable feature in compounds for in vivo use.

In view of its antiviral activity, Compound A recommends itself for the treatment of a variety of diseases caused by viruses, particularly primary and recurrent infections caused by the Herpesviridae in human and non-human animal subjects. Such diseases include stomatitis, skin eruptions, shingles, encephalitis, eye and genital herpes infections, retinitis and pneumonitis.

Thus in a further aspect of the present invention, we provide Compound A for use in medicine, more particularly for use in the therapy or prophylaxis of viral infections, especially Herpesviridae, (e.g. herpes simplex) infections, in a human or non-human animal subject.

According to a further aspect of the invention we provide the use of Compound A for the manufacture of a medicament for the therapy or prophylaxis of viral infections, especially primary and recurrent infections caused by the Herpesviridae in a human or non-human animal subject.

According to a yet further aspect of the invention we provide a method of treatment of the human or non-human animal body to combat viruses, especially the Herpesviridae, which method comprises administering to the body an effective amount of Compound A.

Compound A may be formulated for administration in any convenient way, and the invention therefore also includes within its scope pharmaceutical compositions for use in the therapy or prophylaxis of viral infections, especially Herpesviridae (e.g. herpes simplex) infections, in a human or non-human animal subject comprising Compound A together, if desirable, with one or more physiologically acceptable carriers or excipients.

Compound A may, for example, be formulated for oral, buccal, parenteral, topical or rectal administration.

Tablets and capsules for oral administration may contain conventional excipients such as binding agents, for example syrup, acacia, gelatin, sorbitol, tragacanth, mucilage of starch or polyvinyl pyrrolidone; fillers, for example, lactose, sugar, maize-starch, calcium phosphate or sorbitol; lubricants, for example, magnesium stearate, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives (such as suspending agents), for example, sorbitol syrup, methyl cellulose, glucose/sugar syrup, gelatin,

hydroxymethyl cellulose, carboxymethyl cellulose, aluminium stearate gel or hydrogenated edible fats; emulsifying agents, for example, lecithin, sorbitan monooleate or acacia; non-aqueous vehicles (which may include edible oils), for example almond oil, fractionated coconut oil, oily esters, propylene glycol or ethyl alcohol; or preservatives, for example, methyl or propyl p-hydroxybenzoates or sorbic acid. The compound may also be formulated as suppositories, e.g. containing conventional suppository bases such as cocoa butter or other glycerides.

For buccal administration the compositions may take the form of tablets or lozenges formulated in conventional manner.

Compound A may also be formulated for injection and may be presented in unit dose form, for instance as ampoules, vials, small volume infusions or pre-filled syringes, or in multi-dose containers with an added preservative. The compositions may take such forms as solutions, suspensions, or emulsions in aqueous or non-aqueous vehicles, and may contain formulatory agents such as anti-oxidants, buffers, antimicrobial agents and/or toxicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

For topical administration Compound A may be formulated as ointments, creams, lotions, powders, pessaries, sprays, aerosols or drops (e.g. eye or nose drops). Ointments and creams may, for example, be formulated with an aqueous or oily base with the addition of suitable thickening and/or gelling agents. Such bases may thus, for example, include water and/or an oil such as liquid paraffin or a vegetable oil such as arachis oil or castor oil. Thickening agents which may be used include soft paraffin, aluminium stearate, cetostearyl alcohol, polyethylene glycols, microcrystalline wax and beeswax.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents or suspending agents.

Aerosol sprays are conveniently delivered from pressurised packs, with the use of a suitable propellant, e.g. dichlorodifluoromethane, trichlorofluoromethane, dichlorotetrafluoroethane, carbon dioxide or other suitable gas.

The pharmaceutical compositions according to the invention may also contain other active ingredients such as antimicrobial agents, or preservatives.

The compositions may contain from 0.1%-99% of the active material. For topical administration, for example, the composition will generally contain from 0.01% to 20%, more preferably 0.5% to 5% of the active material.

For topical administration the daily dosage as employed for adult human treatment will range from 0.1 mg to 1000 mg, preferably 0.5 mg to 10 mg. However, it will be appreciated that extensive skin infections may require the use of higher doses.

For systemic administration the daily dosage as employed for adult human treatment will range from 5 mg to 5000 mg, preferably 50 mg to 2000 mg, which may be administered in 1 to 5 daily doses, for example 50 mg to 500 mg. For serious infections the compound may be administered by intravenous infusion using, for example 0.01 to 10 mg/kg/hr of the active ingredient.

Compound A may be administered in combination with one or more further therapeutic agents such as a different antiviral agent.

The invention thus provides, in a further aspect, a combination comprising Compound A together with another therapeutically active agent, in particular an antiviral agent.

The combinations referred to above may conveniently be presented for use in the form of a pharmaceutical formulation and thus pharmaceutical formulations comprising a combination as defined above together with a pharmaceutically acceptable carrier comprise a further aspect of the present invention.

When Compound A is used in combination with a second therapeutic agent active against the same virus the dose of each compound will vary from that when the compound is used alone. Appropriate doses will be readily appreciated by those skilled in the art.

Suitable methods for preparing Compound A are described below.

According to another aspect of the present invention, we provide processes for the preparation of Compound A. Thus, one process (A) for the preparation of Compound A comprises treating the parent of Compound A with a suitable base, for example sodium hydroxide in a suitable solvent (e.g. water). The salt may conveniently be prepared for example, by adding sodium hydroxide to the parent of Compound A in a

suitable solvent such as water at a temperature of, for example, 0°C to room temperature.

The parent of Compound A may conveniently be prepared by hydrolysing a compound of formula (2)

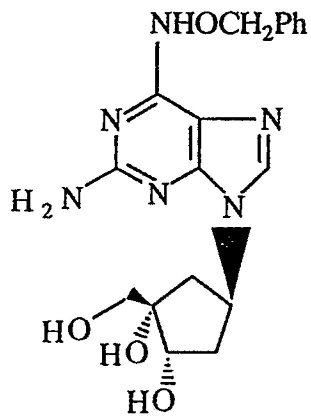

(2)

Hence, in a further aspect of this invention, we provide a process for the preparation of (1'S,3'S,4'S)-2-amino-1,9-dihydro-9-(3,4-dihydroxy-3-hydroxymethyl-1-cyclopentyl)-6H-purin-6-one which comprises hydrolysing a compound of formula (2).

Another process (B) for preparing Compound A comprises hydrolysing a compound of formula (2) followed by treatment of the resulting parent of Compound A with a suitable base as described in process (A) above.

The hydrolysis of the compound of formula (2) to provide the parent of Compound A may conveniently be effected in water or a mixture of water and a suitable water-miscible solvent such as an alcohol (e.g. methanol or ethanol), an ether (e.g. dioxan or tetrahydrofuran), a ketone (e.g. acetone), an amide (e.g. dimethylformamide) or a sulphoxide (e.g. dimethylsulphoxide). The reaction conveniently takes place in the presence of an acid. Suitable acids include organic acids (p-toluenesulphonic acid or glacial acetic acid) and inorganic acids (e.g. mineral acids such as hydrochloric, nitric or sulphuric acid). In some cases the acid may be used as the reaction solvent, especially when the acid used is hydrochloric acid. Hydrolysis is conveniently effected at a temperature in the range of -10°C to +150°C, e.g. +50°C to +120°C. The hydrolysis is preferably effected by heating a compound of formula (2) in hydrochloric acid, e.g. at reflux.

The hydrolysis step described hereinabove is very convenient, provides the parent of Compound A in good yield and may be suitable for use on an industrial scale. The hydrolysis of the compound of formula (2) is also more cost effective than the hydrolysis of related alkoxylamines described in European Patent Application Publication No. 0345076.

The compound of formula (2) may be prepared from compounds of formula (3)

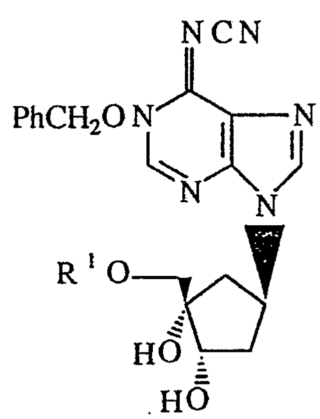

(3)

(wherein $R^1$ is a hydrogen atom or a suitable hydroxyl protecting group as defined below) by heating the compound (3), e.g. at reflux, in a suitable solvent such as an alcohol (e.g. ethanol) or a mixture of water and

an alcohol (e.g. aqueous ethanol) and in the presence of a suitable base (e.g. 1,8-diazabicyclo[5.4.0]undec-7-ene or an alkali metal hydrogen carbonate such as potassium hydrogen carbonate).

Compounds of formula (3) may be prepared from the compound of formula (4)

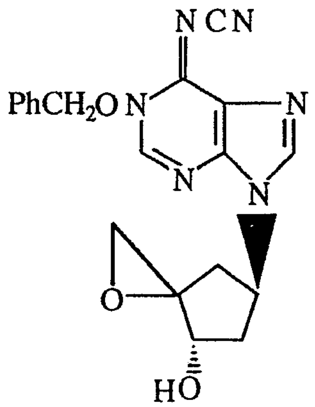

(4)

by reaction with $R^1OH$ (where $R^1$ is a suitable hydroxyl protecting group as defined below) under epoxide ring opening conditions, followed if necessary by removal of the hydroxyl protecting group.

When $R^1$ represents a hydroxyl protecting group $R^1$ includes any suitable hydroxyl protecting group, for example, as described in 'Protective Groups in Organic Chemistry', Ed. J. F. W. McOmie (Plenum Press 1973) or 'Protective Groups in Organic Synthesis' by Theodora W. Greene (John Wiley and Sons, 1981). Examples of suitable hydroxyl protecting groups include groups selected from alkyl (e.g. methyl or t-butyl), aralkyl (e.g. benzyl) or acyl (e.g. acetyl or benzoyl). The hydroxyl protecting groups may be removed by conventional techniques. Thus, for example, alkyl and acyl groups may be removed by solvolysis, e.g. by hydrolysis under acidic or basic conditions. Aralkyl groups such as benzyl may be cleaved by hydrogenolysis in the presence of a nobel metal catalyst such as palladium-on-charcoal.

When $R^1$ represents an acyl group such as benzoyl the epoxide ring opening reaction to provide a compound of formula (3) may conveniently be effected in the presence of an alkali metal salt (e.g. the sodium salt) of the acid $R^1OH$ and in a suitable solvent such as an amide (e.g. N,N-dimethylformamide) at an elevated temperature (e.g. about 50°C), preferably in the presence of a crown ether such as 15-crown-5.

The compound of formula (4) may be prepared from the compound of formula (5)

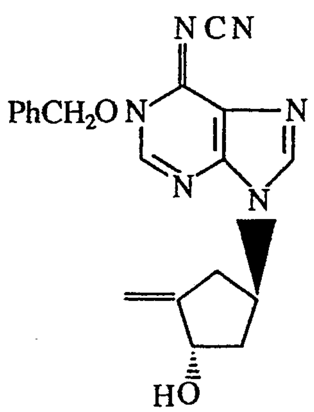

(5)

by treatment with tert-butyl hydroperoxide conveniently in the presence of a vanadium catalyst such as vanadyl acetylacetonate in a suitable solvent such as a halogenated hydrocarbon (e.g. dichloromethane) at an elevated temperature, e.g. reflux.

The compound of formula (5) may be prepared from the compound of formula (6)

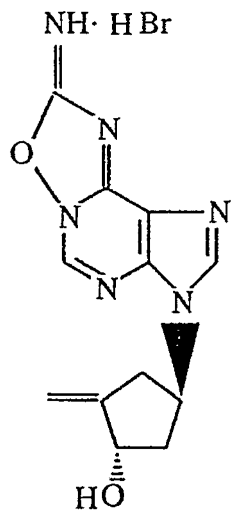

(6)

by adding a suitable base such as a tertiary amine (e.g. triethylamine) to a solution of the compound (6) and a benzyl halide (e.g. benzyl bromide) in a solvent such as an amide (e.g. dimethylformamide) and allowing the reaction to proceed at about ambient temperature.

The compound of formula (6) may be prepared from the compound of formula (7)

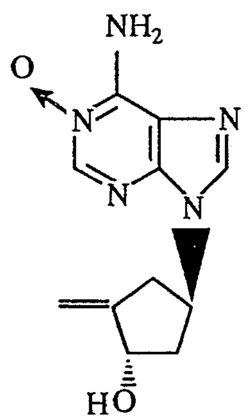

(7)

by reaction with cyanogen bromide in a solvent such as an alcohol (e.g. methanol) at reduced temperature, e.g. $-50^0$ to $0^0C$.

The compound of formula (7) may be prepared from the compound of formula (8)

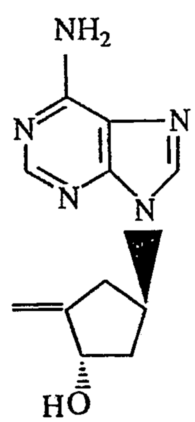

(8)

by oxidation, for example using a peracid such as m-chloroperbenzoic acid. The reaction is conveniently effected in a suitable solvent such as an alcohol (e.g. ethanol) at about ambient temperature.

The compound of formula (8) may be prepared from compounds of formula (9)

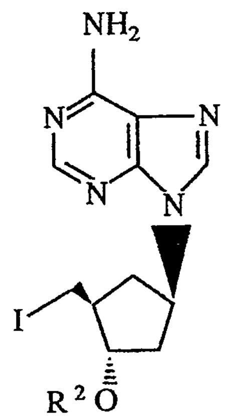

(9)

(wherein $R^2$ is a suitable acyl group as defined below) by heating the compound (9), e.g. at a temperature of about 80°C in a suitable solvent such as pyridine and optionally in the presence of a base (e.g. 1 ,5-diazabicyclo[4.3.0]non-5-ene), followed by removal of the protecting group $R^2$. $R^2$ represents an acyl group such as benzoyl which may be removed using an ion exchange resin such as Amberlite 1RA 400(OH).

Compounds of formula (9) may be prepared by iodination of a compound of formula (10)

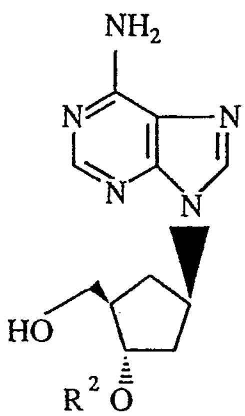

(10)

(wherein $R^2$ is as defined previously). Suitable iodinating agents include phosphonium iodides (e.g. methyl triphenoxyphosphonium iodide) and the iodination reaction may conveniently be effected at a temperature in the range of -70°C to +20°C in a suitable solvent such as an ether (e.g. tetrahydrofuran) or an amide (e.g. dimethylformamide).

Compounds of formula (10) may be prepared from the known compound aristeromycin (11)

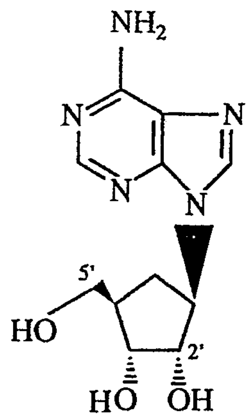

(11)

by conversion of the 2′-α-OH group to a hydrogen atom and the introduction of the $R^2$ protecting group. This may conveniently be achieved by initially protecting the aristeromycin 5′-OH group, for example as a silyl ether by reaction with a silyl halide such as a trialkylsilyl halide (e.g. t-butyldimethylsilyl chloride or thexyldimethylsilyl chloride). The $R^2$ protecting group may then conveniently be introduced by treating the silylated intermediate with n-dibutyltin oxide and then with an acyl halide $R^2$Hal (where Hal is a halogen atom, e.g. chlorine). Subsequent conversion of the 2′-α- OH group to a hydrogen atom may be effected by converting the hydroxyl group to a leaving group removable by reduction (e.g. by homolytic reduction) and thereafter reducing the aforesaid compound using, for example, an alkyltin hydride (e.g. tri-n-butyltin hydride) in the presence of a radical initiator such as a peroxide, 2,2′-azobis(2-methylpropionitrile) or light. Suitable leaving groups include -OC(=S)OR$^3$ (where $R^3$ is $C_{1-6}$alkyl, aryl such as phenyl, heteroaryl such as imidazole or $C_{1-6}$alkylaryl such as p-tolyl). Conversion of the hydroxyl group to -OC(=S)OR$^3$ may be effected using, for example, aryl halothionoformates such as phenyl chlorothionocarbonate in the presence of a suitable base such as an amine (e.g. 4-dimethylaminopyridine) and in a solvent such as a halogenated hydrocarbon (e.g. dichloromethane).

Alternative processes for preparing the parent of Compound A are described in our European Patent Application Publication No. 0345076.

Intermediates of formulae (2) to (9) are novel compounds and form further aspects of the present invention. The compound of formula (2) is a particularly useful intermediate in the synthesis of Compound A from aristeromycin.

It should be understood that the individual steps in the process herein for preparing Compound A from aristeromycin and sequential combinations of such steps including the overall multistep process represent particular aspects of this invention.

The following Preparations and Examples illustrate the present invention but should not be construed as a limitation of the invention.

Intermediate 1

(1R,2S,3R,5R)-3-[6-Amino-9H-purin-9-yl]-5-[((2,3-dimethylbut2-yl)-dimethylsilyloxy)methyl]-1,2-cyclopentanediol

A mixture of aristeromycin (50g) and dimethylformamide (250ml) was stirred at room temperature and imidazole (50g) and then dimethylthexylsilyl chloride (10ml) were added. Three further 10ml portions of dimethylthexylsilyl chloride were added after 20min, 1h and 1.75h. After 4h the resulting mixture was poured into rapidly stirred water (2.5L). The suspension was filtered and the residue washed with water (2 x 250ml). The air dried solid was added to di-isopropyl ether (500ml) and stirred for 20 min. The suspension was filtered and the residue collected, stirred with diethyl ether (500ml) and filtered to give the title compound (49g); $^1$H nmr (DMSO-$d_6$) δ8.13 (1H), 8.09 (1H), 7.18 (2H), 5.03-4.93 (1H), 4.76-4.60 (2H), 4.41-4.28 (1H), 3.88-3.78 (1H), 3.72-3.57 (2H), 2.28-1.70 (3H), 1.67-1.52 (1H), 0.95-0.75 (12H), 0.1 (6H).

Intermediate 2

(1R,2S,3R,5R)-3-[6-Amino-9H-purin-9-yl]-5-[((2,3-dimethylbut-2-yl)-dimethylsilyloxy)methyl]-1,2-cyclopentanediol, benzoate

A stirred mixture of Intermediate 1(225 g) and n-dibutyltin oxide (139 g) in toluene (2 L) and methanol (200 ml) was heated under reflux for 1.25 h and then distilled for 0.5 h. The resulting mixture was allowed to cool for a while before being filtered, whilst still hot. The filtrate was evaporated to a solid (371 g).

A solution of benzoyl chloride (66 ml) in chloroform (60 ml) was added over 20 min to a stirred and cooled (ice-water bath) solution of the above solid (371 g) and triethylamine (86ml) in chloroform (1.8 L). During the next two hours, benzoyl chloride (10 ml) was added in portions. Then, a saturated aqueous solution of sodium bicarbonate (650 ml) was added. After 10 min, the resulting mixture was filtered through a pad of Hyflosupercel. The residual solids were washed thoroughly with chloroform (1 L). The phases were separated and the organic phase was washed successively with a saturated aqueous solution of sodium bicarbonate (1 L) and water (2 x 600 ml). The combined aqueous phases were extracted with chloroform. The combined organic phases were dried over magnesium sulphate and evaporated to a solid, which was heated in boiling ethyl acetate (800 ml). After 10 min, the resulting slurry was allowed to cool and then filtered. The collected solid was washed successively with ethyl acetate (200 ml) and di-isopropyl ether (400 ml). Drying gave the title compound as a white solid (200 g); $\lambda_{max}$ (ethanol) 230 nm($E^{1\%}_{1cm}$318),26.8nm($E^{1\%}_{1cm}$307); $^1$H nmr(DMSO-$d_6$) $\delta$ 8.19 (1H), 8.12 (1H), 8.09-9.02 (2H), 7.72-7.63 (1H), 7.60-7.50 (2H), 7.22 (2H), 5.51-5.45 (1H), 5.36-5.27 (1H), 4.90-4.65 (2H), 3.84-3.67 (2H), 2.46-1.95 (3H), 1.66-1.52 (1H), 0.86 (3H), 0.84 (9H), 0.11 (6H).

Intermediate 3
(1R,2S,3R,5R)-3-(6-Amino-9H-purin-9-yl)-5-[((2,3-dimethylbut-2-yl) dimethylsilyloxy)methyl]-1,2-cyclopentanediol, 1-benzoate-2-phenoxythiocarboxylate

A solution of phenyl chlorothionoformate (73 ml) in dichloromethane (200 ml) was added dropwise over 50 min to a stirred and cooled (ice-water bath) suspension of Intermediate 2 (225g) and 4-dimethylaminopyridine (107 g) in dichloromethane (2 L). After 30 min, the resulting yellow solution was washed successively with saturated aqueous sodium bicarbonate solution (600 ml), 1M-hydrochloric acid (2 x 600 ml), water (2 x 600 ml) and saturated aqueous sodium bicarbonate solution (600 ml and 300 ml). The organic phase was dried over dried magnesium sulphate and then evaporated to a yellow foam. Diethyl ether (350 ml) was added, with heating, followed by a slow addition of petroleum ether (b.p. 40-60$^0$C) (1 L), which resulted in the displacement of a second phase, as a syrup which soon solidified. The solid was crushed to a powder and the mixture filtered. The collected solid was washed with petroleum ether (500 ml) and dried to give the title compound as a white solid (275 g); $\lambda_{max}$ (ethanol) 232.2 nm ($E^{1\%}_{1cm}$339), 258.8 nm ($E^{1\%}_{1cm}$272); $^1$H nmr (DMSO-$d_6$) $\delta$ 8.29 (1H), 8.14 (1H) 8.11-8.03 (2H), 7.77-7.68 (1H), 7.65-7.55 (2H), 7.38-7.15 (5H), 6.91-6.83 (2H), 6.37-6.28 (1H), 5.83-5.76 (1H), 5.49-5.34 (1H), 3.94-3.74 (2H), 2.68-2.28 (3H), 1.68-1.54(1H), 0.86 (3H), 0.85 (3H), 0.84 (6H), 0.13 (6H).

Intermediate 4
(1S,2R,4R)-4-[6-Amino-9H-purin-9-yl]-2-[((2,3-dimethylbut-2-yl) dimethylsilyloxy)methyl]-cyclopentanol, benzoate

Nitrogen was passed through a stirred solution of Intermediate 3(275 g), tri-n-butyltin hydride (160 ml) and 2,2′-azobis-(2-methylpropionitrile) (12g) in toluene (2 L) for 45 min. The solution was then heated (oil-bath at 75 ± 5$^0$C). After 2.25 h, the solution was cooled and evaporated to a yellow syrup. Di-isopropyl ether (500 ml) was added, followed by petroleum ether (b.p. 40-60$^0$C) (650 ml). After 2 h, more petroleum ether (200 ml) was added and the mixture left at 4$^0$C for 16 h. Filtration then gave the title compound as a white crystalline solid (148 g); m.p. 140- 143$^0$C; $\lambda_{max}$ (ethanol) 229.8 nm ($E^{1\%}_{1cm}$328), 261.2 nm ($E^{1\%}_{1cm}$320); $^1$H nmr (DMSO-$d_6$) $\delta$ 8.22 (1H), 8.12 (1H), 8.06-7.97 (2H), 7.72-7.63 (1H), 7.61-7.50 (2H), 7.22 (2H), 5.42-5.33 (1H), 5.18-5.01(1H), 3.86-3.69 (2H), 2.73-1.94 (5H), 1.63-1.49 (1H), 0.83(3H), 0.80 (9H), 0.09 (6H).

Intermediate 5
(1S,2R,4R)-4-(6-Amino-9H-purin-9-yl)-2-hydroxymethylcyclopentanol, benzoate

A solution of tetra-n-butylammonium fluoride in tetrahydro- furan (1 molar) (620 ml) was added to a stirred solution of Intermediate 4(218 g) in tetrahydrofuran (1.4 L). After 1.5 h ethanol (100 ml) was added. After a further 30 min, the solution was evaporated to a slurry which was filtered. The collected solid was washed successively with ethanol (150 ml), ethanol - di-isopropyl ether (1:1) (240 ml) and di-isopropyl ether (200 ml). Drying gave the title compound as a white solid (114 g), m.p. 206-210$^0$C, $\lambda_{max}$ (ethanol) 229.8 nm ($E^{1\%}_{1cm}$430), 260.8 nm ($E^{1\%}_{1cm}$429); $^1$H nmr (DMSO-$d_6$) $\delta$8.28 (1H), 8.14 (1H), 8.07-7.97 (2H), 7.75-7.64 (1H),

7.62-7.50 (2H), 7.22 (2N), 5.42-5.13 (1H), 5.19-5.01 (1H), 4.89 (1H), 3.70-3.55 (2H), 2.71-1.91 (5H).

Intermediate 6
(1S,2S,4R)-4-(6-Amino-9H-purin-9-yl)-2-iodomethyl cyclopentanol, benzoate

Methyltriphenoxyphosphonium iodide (219 g) was added over 5 min to a stirred and cooled (acetone-drikold bath) suspension at -62$^{0}$C of Intermediate 5 (114 g) in tetrahydrofuran (2 L). After 12 min, the cooling bath was removed and the mixture was stirred at ambient temperature. After 2 h, methanol (60 ml) was added and after a further 10 min, the resulting mixture was filtered through a pad of Hyflosupercel. The filtrate was evaporated to a brown syrup, which was stirred with diethyl ether (1 L). After 45 min, the mixture was filtered. The collected solid was stirred with di-isopropyl ether (300 ml). Methanol (300 ml) was then added. After a few min, the mixture was filtered and the collected solid was washed successively with a mixture of di-isopropyl ether - methanol (1:1) (400 ml), di-isopropyl ether (200 ml) and diethyl ether (200 ml). Drying gave the title compound as a pale yellow solid (84 g); $\lambda_{max}$ (methanol) 230.4 nm ($E^{1\%}_{1c}$ $_{m}$371), 261.0 nm ($E^{1\%}_{1c}$ $_{m}$ 366); $^{1}$H nmr (DMSO-d$_6$) $\delta$ 8.28 (1H), 8.15 (1H), 8.08-7.78 (2H), 7.75-7.65 (1H), 7.63-7.51 (2H), 7.29 (2H), 5.33-5.23 (1H), 5.21-5.05 (1H), 3.67-3.50 (2H), 2.83 - ca. 1.95 (5H).

Intermediate 7
(1S,4R)-4-(6-Amino-9H-purin-9-yl)-2-methylenecyclopentanol, benzoate

Nitrogen was passed through a solution of Intermediate 6 (163.5g) in pyridine (1.4 L) for 20 min. The solution was then stirred and a solution of 1,5-diazabicyclo[4.3.0]non- 5-ene (49 ml) in pyridine (50 ml) was added. The resulting black solution was heated (oil-bath at 80 ± 3$^{0}$C). After 1 h, the resulting solution was allowed to cool and then evaporated to a syrup, which was dissolved in chloroform (600 ml). The resulting solution was washed successively with water (2 x 600 ml) and saturated aqueous sodium bicarbonate solution (2 x 300 ml) and then dried over magnesium sulphate. Evaporation gave a black syrup which was purified by chromatography on silica-gel. Elution was by mixtures of chloroform and methanol (80:1 to 20:1). Combination of appropriate fractions and then evaporation gave a foam which was crystallised from a mixture of dichloromethane and di-isopropyl ether to give the title compound as a white solid (88.5 g); m.p. 146-148$^{0}$C; $\lambda_{max}$ (ethanol) 230.2 nm ($E^{1\%}_{1c}$ $_{m}$467), 261.0 nm ($E^{1\%}_{1c}$ $_{m}$469); $^{1}$H nmr (DMSO-d$_6$) $\delta$ 8.28 (1H), 8.15 (1H), 8.07-7.94 (2H), 7.74-7.64 (1H), 7.62-7.51 (2H), 7.24 (2H), 5.96-5.88 (1H), 5.42 (1H), 5.32 (1H), 5.29-5.14 (1H), 3.20-2.92 (2H), 2.86-2.40 (2H).

Intermediate 8
(1S,4R)-(6-Amino-9H-purin-9-yl)-2-methylenecyclopentanol

A stirred mixture of Intermediate 7 (88.5 g) and Amberlite 1RA 400 (OH) (280 g) in methanol (900 ml) was heated under reflux. After 1.75 h the mixture was allowed to cool and then filtered. The filtrate was reduoed in volume and then filtered through a pad of Hyflosupercel. The filtrate was evaporated to a syrup, which was crystallised from a mixture of methanol (a few ml) and ethyl acetate (200 ml). Filtration gave the title compound as a white solid (54.6 g); m.p. 158-163$^{0}$C; $\lambda_{max}$ (methanol) 260.6 nm ($E^{1\%}_{1c}$ $_{m}$601); $^{1}$H nmr (DMSO-d$_6$) $\delta$ 8.20 (1H), 8.12 (1H), 7.21 (2H), 5.19 (1H), 5.13 (1H), 5.1-5.0 (1H) 5.06 (1H), 4.63-4.53 (1H), 3.04-2.74 (2H), 2.48-2.02 (2H).

Intermediate 9
(1S,4R)-4-(6Amino-9H-purin-9-yl)-2-methylenecyclopentanol, 1′-oxide

m-Chloroperbenzoic acid (80%) (63.53 g) was added to a stirred suspension of Intermediate 8(54.5 g) in ethanol (1 L). After 15 min, ethanol (200 ml) was added. After a further 15 min, more ethanol (400 ml) was added. After a further 1.25 h, water (300 ml) was added. The resulting solution was filtered and the filtrate evaporated to about 300 ml. Water (200 ml) was added and evaporation of ethanol was continued. The resulting mixture was filtered and the filtrate was evaporated to a white solid which was stirred with ethyl acetate (200 ml). Filtration gave the title compound as a white solid (38g); $\lambda_{max}$ (water) 232.2 nm ($E^{1\%}_{1c}$ $_{m}$1398), 262.0 nm ($E^{1\%}_{1c}$ $_{m}$285); $^{1}$H nmr (DMSO-d$_6$) $\delta$ 8.62 (1H), 8.18 (1H), 5.19 (1H), 5.07 (1H), 5.25-5.01 (1H), 4.62-4.52 (1H), 3.07-2.73 (2H), 2.47-2.05 (2H).

Intermediate 10
(1S,4R)-4-(2-Imino-1,2-dihydro-[1,2,4]oxadiazolo[3,2-i]purin-7-yl)-2-methylenecyclopentanol, hydrobromide

A solution of cyanogen bromide (27.5 g) in methanol (200 ml) was added over 15 min to a stirred and cooled (ice-water bath) suspension of Intermediate 9 (49.56 g) in methanol (500 ml). After 2.25 h the resulting yellow solution was evaporated. Diethyl ether (300 ml) was added and the mixture stirred. After 5 h, the diethyl ether was decanted and the solid was washed with more diethyl ether. Drying gave the title compound as a pale yellow solid (66.3 g); $\lambda_{max}$ (ethanol) 227.2 nm ($E^{1\%}_{1cm}$531), 286.2 nm ($E^{1\%}_{1cm}$517); $^1H$ nmr ($DMSO-d_6$) $\delta$ 10.75-10.45 (2H), 10.06 (1H), 8.94 (1H), 5.38-5.23 (1H), 5.24 (1H), 5.01 (1H), 4.66-4.55 (1H), 3.15-2.76 (2H), 2.5-2.16 (2H).

Intermediate 11

(1S,4R)-4-(6-Cyanoimino-1,6-dihydro-1-phenylmethoxy-9H-purin-9-yl)-2-methylenecyclopentanol

Triethylamine (52 ml) was added to a stirred and cooled (ice-water bath) solution of Intermediate 10 (44 g) and benzyl bromide (44 ml) in dimethylformamide (300 ml). After 10 min, the cooling bath was removed. After 2 h, the resulting yellow solution was poured into water (2.5 L) and the pH adjusted to 7 with sodium bicarbonate. The mixture was extracted with chloroform. The organic phase was washed with water (2 x 300 ml) and dried over magnesium sulphate. Evaporation gave a slurry, which was stirred and di-isopropyl ether (500 ml) was added. The mixture was filtered and the solids washed with di-isopropyl ether. Drying gave the title compound as a yellow solid (36.1 g); $\lambda_{max}$ (ethanol) 286.6 nm ($E^{1\%}_{1cm}$494); $^1H$ nmr ($DMSO-d_6$) $\delta$ 1cm 8.76 (1H), 8.48 (1H), 7.64-7.54 (2H), 7.50-7.39 (3H), 5.33 (2H), 5.20 (1H), 5.08 (1H), 5.18 (1H), 5.17-5.05 (1H), 4.62-4.52 (1H), 3.08-2.95 (1H), 2.84-2.69 (1H), 2.43-2.07 (2H).

Intermediate 12

(3S,4S,6R)-6-(6-Cyanoimino-1,6-dihydro-1-phenylmethoxy-9H-purin-9-yl)-1-oxaspiro[2.4]heptan-4-ol

A solution of t-butyl hydroperoxide in 2,2,4-trimethyl- pentane (3 molar) (42 ml) was added to a stirred mixture of Intermediate 11 (35 g) and vanadyl acetylacetonate (3 g) in dichloromethane (600 ml). The resulting solution was heated under reflux. During the next 1.75 h, more vanadyl acetylacetonate (1.5 g) was added. After a further 1 h, the mixture was allowed to cool and then filtered. The filtrate was evaporated to a syrup. Diethyl ether (500 ml) was added and the mixture stirred. Filtration gave a yellow solid, which was washed with diethyl ether and dried to give the title compound as a yellow solid (35.9 g); $\lambda_{max}$ (ethanol) 287.2 nm ($E^{1\%}_{1cm}$361); $^1H$ nmr ($DMSO-d_6$) $\delta$ 8.78 (1H), 8.53 (1H), 7.67-7.53 (2H), 7.53-7.38 (3H), 5.33 (2H), 5.37-5.22 (1H), 5.01 (1H), 4.18-4.08 (1H), 2.92 (2H), 2.5-2.17 (4H).

Intermediate 13

(1S,2S,4S)-4-(6-Cyanoimino-1,6-dihydro-1-phenylmethoxy-9H-purin-9-yl)-1,2-dihydroxycyclopent-1-ylmethyl, benzoate

1,4,7,10,13-Pentaoxacyclopentadecane (22.7 ml) was added to a stirred mixture of Intermediate 12 (36g), benzoic acid (23.2 g) and sodium benzoate (27.4 g) in N,N-dimethylformamide (250 ml). The mixture was heated (oil-bath at 55 ± 5$^0$C). After 1.75 h the mixture was allowed to cool and then filtered. The filtrate was evaporated to a syrup which was mixed with ethyl acetate then washed with aqueous sodium bicarbonate solution and dried over magnesium sulphate. Evaporation gave a foam which was stirred in boiling chloroform (150 ml). After cooling, filtration gave the title compound as a white solid (15.7 g); $\lambda_{max}$ - (methanol) 224.8 nm ($E^{1\%}_{1cm}$653), 286.8 ($E^{1\%}_{1cm}$384); $^1H$ nmr ($DMSO-d_6$) $\delta$ 8.73 (1H), 8.54 (1H), 8.07-7.98 (2H), 7.73-7.64 (1H), 7.63-7.40 (7H), 5.32 (2H), 5.27-5.11 (1H), 5.15 (1H), 4.93 (1H), 4.47 (1H), 4.30 (2H), 2.40-2.20 (4H).

Intermediate 14

(1S,2S,4S)-4-(2-Amino-6-phenylmethoxyamino-9H-purin-9-yl)-1-hydroxymethyl-1,2-cyclopentanediol

Potassium hydrogen carbonate (0.84 g) was added to a stirred mixture of Intermediate 13 (4.2g) in ethanol (80 ml) and water (8 ml), which was then heated under reflux. After 2.5 h the resulting solution was allowed to cool and then evaporated to a foam. Purification by chromatography on silica-gel, with elution by chloroform - methanol mixtures (10:1 to 1:1) gave thetitle compound as a white foam (2.14g); $\lambda_{max}$ - (methanol) 281.2nm ($E^{1\%}_{1cm}$419); $^1H$ nmr ($DMSO-d_6$) $\delta$ 9.78 (1H), 7.57 (1H), 7.47-7.23 (5H), 6.56 (2H), 5.00 (2H), 4.90-4.05 (5H), 3.17 (2H), 2.10-1.95 (4H).

Intermediate 15
(1'S,3'S,4'S)-2-Amino-1,9-dihydro-9-(3,4-dihydroxy-3-hydroxymethyl-1-cyclopentyl)-6H-purin-6-one

A solution of Intermediate 14 (2 g) in 3M-hydrochloric acid (30 ml) was stirred and heated under reflux. During the next 2.5 h, 6M-hydrochloric acid (3 ml) was added in two portions. After a further 1.5 h, the solution was allowed to cool and then evaporated. The residual solid was treated with aqueous sodium hydroxide solution (1 molar) until the pH was 6-7. Filtration gave a solid, which was washed with water, and then recrystallised from boiling water to give the title compound as a white solid (0.71 g); m.p. 168-176°C; $\lambda_{max}$ (pH 6 buffer) 253.2 nm ($E^{1\%}_{1cm}$428); $^1$H nmr (DMSO-$d_6$) $\delta$ 10.54 (1H), 7.79 (1H), 6.41 (2H), 4.91 (1H), 4.81-4.72 (2H), 4.25 (1H), 4.21 (1H), 3.45-3.25 (2H), 2.16-1.98 (4H).

Example 1
(1'S,3'S,4'S)-2-Amino-1,9-dihydro-9-(3,4-dihydroxy-3-hydroxy-methyl-1-cyclopentyl)-6H-purin-6-one, sodium salt

An aqueous solution of sodium hydroxide (0.1 molar) (21.97 ml) was added to a stirred suspension of Intermediate 15 (0.62 g) in water (5 ml). The resulting solution was freeze-dried to give the title compound as a solid (0.72 g); $\lambda_{max}$ (pH 6 buffer) 252.4 nm ($E^{1\%}_{1cm}$392); $^1$H nmr (DMSO-$d_6$) $\delta$ 7.51 (1H), 4.89 (1H) 4.22 (1H), 2.17-1.95 (4H).

Example 2
Pharmaceutical compositions

| (1) Topical creams | |
|---|---|
| | % w/v |
| a) Sodium salt of the active ingredient | 0.25 |
| b) Butylene glycol | 15.0 |
| c) Glycerol | 2.5 |
| d) Cetostearyl alcohol | 10.0 |
| e) Self emulsifying monostearin | 1.5 |
| f) Polyoxyethylene (2) oleyl ether | 5.0 |
| g) Beeswax | 3.0 |
| h) Chlorocresol | 0.1 |
| Distilled water | to 100.0 |

Heat the water to 70$^0$ and dissolve the chlorocresol (h). Melt (d), (e), (f) and (g) together, heating to 70$^0$. Add the melt to the water with stirring. Disperse (a) in a mixture of (b) and (c) and add the dispersion (warmed to 55$^0$) to the bulk mixture. Cool, with stirring, to 35$^0$.

| (2) Eye Ointment | |
|---|---|
| | % w/v |
| Sodium salt of the active ingredient | 3.0 |
| Liquid paraffin | 25.00 |
| White soft paraffin | to 100.0 |

Melt the white soil paraffin by heating to 70$^0$. Disperse the sodium salt of the active ingredient in the liquid paraffin, warm the dispersion to 55$^0$ and add it with stirring to the molten white soft paraffin. Cool, with stirring, to 35$^0$.

| (3) Eye Drops | |
|---|---|
| | % w/v |
| Sodium salt of the active ingredient | 0.5 |
| Benzalkonium chloride | 0.01 |
| Sodium chloride | 0.85 |
| Water for injections | to 100.0 |

Dissolve the benzalkonium chloride, sodium chloride and the sodium salt of the active ingredient in the water. Filter the solution, collect the filtrate aseptically and fill (aseptically) into suitable sterile eye drop containers.

| (4a) Oral Tablet | | |
|---|---|---|
| | mg/Tablet | % w/w |
| Sodium salt of the active ingredient | equivalent to 100mg active ingredient | 40.1 |
| Lactose | 100mg | 37.2 |
| Maize starch | 50 | 18.6 |
| Polyvinyl pyrrolidone | 2 | 0.75 |
| Sodium starch glycolate | 7 | 2.6 |
| Magnesium stearate | 2 | 0.75 |

Sieve the sodium salt of the active ingredient and maize starch through a 40 mesh screen. Blend the maize starch with the lactose and the sodium salt of the active ingredient in a suitable blender. Make an aqueous solution of the polyvinyl pyrrolidone in a 5-10% w/v solution. Add this solution to the mixing powders and mix until granulated. Using suitable equipment pass the granulate through a 12 mesh screen. Dry the granules in an oven or in a fluid bed dryer. Screen the dry granules through a 16 mesh screen, and blend in the sodium starch glycolate and magnesium stearate previously sieved through a 60 mesh screen. Compress on appropriate punches on an automatic tablet machine. The tablets may be covered in a thin polymer coat applied by the usual film coating techniques. A pigment may be included in the film coat.

| (4b) Oral Tablet | | |
|---|---|---|
| | mg/tablet | % w/w |
| Sodium salt of the active ingredient | equivalent to 100mg active ingredient | 36.1 |
| Microcrystalline cellulose | 183mg | 61.2 |
| Sodium starch glycolate | 6mg | 2.0 |
| Magnesium stearate | 2mg | 0.7 |

Sieve the sodium salt of the active ingredient and microcrystalline cellulose through a 40 mesh screen. Sieve the sodium starch glycolate and magnesium stearate through a 60 mesh screen. Blend the powders together in a suitable blender until homogenous. Compress on appropriate punches on an automatic tablet machine. The tablets may be covered in a thin polymer coat applied by the usual film coating techniques. A pigment may be included in the film coat.

| (5) Oral Capsule | | |
|---|---|---|
| | mg/Capsule | % w/w |
| Sodium salt of active ingredient | equivalent to 100mg active ingredient | 43.3 |
| Lactose anhydrous | 126mg | 50.7 |
| Magnesium stearate | 2mg | 0.8 |
| Sodium starch glycolate | 13mg | 5.2 |

Sieve all the ingredients and mix in a suitable blender. Fill into suitable size hard gelatin capsules using an automatic capsule filling machine.

| (6) Oral syrup | |
|---|---|
| | % w/v |
| Sodium salt of active ingredient | 1.0 |
| Sucrose | 60.0 |
| Colour (optional) | as required |
| Flavour (optional) | as required |
| Distilled water | to 100.0 |

Dissolve the sucrose in water with the aid of heat. Cool and dissolve the sodium salt of the active ingredient and other items. Make up to volume. Fill the solution into suitable syrup containers.

| (7) Powder(for external application) | |
|---|---|
| | % w/w |
| Sodium salt of the active ingredient | 3.0 |
| Silicon dioxide | 2.0 |
| Maize starch | to 100.0 |

Blend the sieved sodium salt of the active ingredient, silicon dioxide and the maize starch in a suitable mechanical blender. Fill the resultant powder blend into suitable powder containers.

In the above pharmaceutical examples the active ingredient is (1′S, 3′S, 4′S)-2-amino-4,9-dihydro-9-[3,4-dihydroxy-3-hydroxymethyl-1-cyclopentyl]-6H-purin-6-one (the parent of Compound A).

## Claims

1. (1′S,3′S,4′S)-2-Amino-1,9-dihydro-9-(3,4-dihydroxy-3-hydroxymethyl-1-cyclopentyl)-6H-purin-6-one, sodium salt (Compound A).

2. A pharmaceutical composition for use in the therapy or prophylaxis of viral infections in a human or non-human animal subject comprising the compound of Claim 1 together with one or more physiologically acceptable carriers or excipients.

3. A composition according to Claim 2 formulated for oral, buccal, parenteral, topical or rectal administration.

4. A composition according to Claim 2 or Claim 3 in a unit dose form.

5. A process for the preparation of the of Claim 1 comprising:

(a) treating (1′S,3′S,4′S)-2-amino-1,9-dihydro-9-(3,4-dihydroxy-3-hydroxymethyl-1-cyclopentyl)-6H-purin-6-one with a suitable base; or

(b) hydrolysing a compound of formula (2)

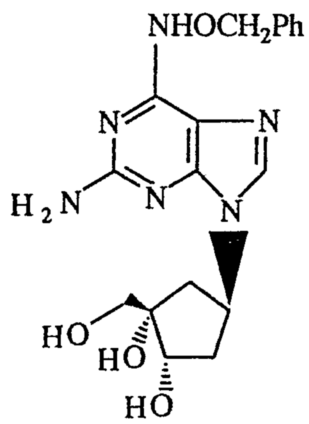

(2)

followed by treatment of the resulting compound (1'S,3'S,4'S)-2-amino-1,9-dihydro-9-(3,4-dihydroxy-3-hydroxymethyl-l-cyclopentyl)-6H-purin-6-one with a suitable base.

6. A process for the preparation of the compound (1'S,3'S,4'S)-2-amino-1,9-dihydro-9-(3,4-dihydroxy-3-hydroxymethyl-1-cyclopentyl)-6H-purin-6-one comprising hydrolysing a compound of formula (2) (as defined in Claim 5).

7. The compound of formula (2).

8. Compounds of formulae (3) to (9).

9. Compound A for use in medicine.

10. Compound A for use in the therapy or prophylaxis of viral infections in a human or non-human animal subject.

11. The use of Compound A for the manufacture of a medicament for the therapy or prophylaxis of Herpesviridae infections in a human or non-human animal subject.

Claims for the following Contracting State: GR

1. A process for the preparation of (1'S,3'S,4'S)-2-Amino-1,9-dihydro-9-(3,4-dihydroxy-3- hydroxymethyl-1-cyclopentyl)-6H-purin-6-one, sodium salt (Compound A), which comprises at least one of the following steps:

(a) treating (1'S,3'S,4'S)-2-amino-1,9-dihydro-9-(3,4-dihydroxy-3-hydroxymethyl-1-cyclopentyl)-6H-purin-6-one with a suitable base; or

(b) hydrolysing a compound of formula (2)

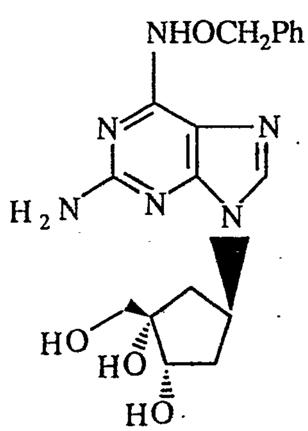

(2)

followed by treatment of the resulting compound (1'S,3'S,4'S)-2-amino-1,9-dihydro-9-(3,4-dihydroxy-3-hydroxymethyl-1-cyclopentyl)-6H-purin-6-one with a suitable base.

2. A process for the preparation of the compound (1'S,3'S,4'S)-2-amino-1,9-dihydro-9-(3,4-dihydroxy-3-hydroxymethyl-1-cyclopentyl)-6H-purin-6-one comprising hydrolysing a compound of formula (2) (as defined in Claim 1).

3. A process according to Claim 1 or Claim 2 wherein the hydrolysis is effected in the presence of an acid.
4. A process according to Claim 1 or Claim 2 wherein the hydrolysis is effected in the presence of hydrochloric acid.
5. A process according to Claim 4 wherein the hydrolysis is effected under reflux.
6. The compound of formula (2).
7. Compounds of formulae (3) to (9).
8. The use of Compound A for the manufacture of a medicament for the therapy or prophylaxis of Herpesviridae infections in a human or non-human animal subject.
Claims for the following contracting State: ES

1. A process for the preparation of (1′S,3′S,4′S)-2-Amino-1,9-dihydro-9-(3,4-dihydroxy-3- hydroxymethyl-1-cyclopentyl)-6H-purin-6-one, sodium salt (Compound A), which comprises at least one of the following steps:
(a) treating (1′S,3′S,4′S)-2-amino-1,9-dihydro-9-(3,4-dihydroxy-3-hydroxymethyl-1-cyclopentyl)-6H-purin-6-one with a suitable base; or
(b) hydrolysing a compound of formula (2)

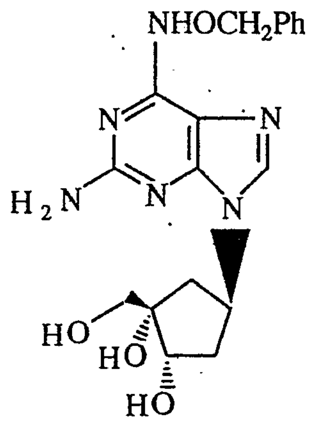

(2)

followed by treatment of the resulting compound (1′S,3′S,4′S)-2-amino-1,9-dihydro-9-(3,4-dihydroxy-3-hydroxymethyl-1-cyclopentyl)-6H-purin-6-one with a suitable base.
2. A process for the preparation of the compound ((1′S,3′S,4′S)-2-amino-1,9-dihydro-9-(3,4-dihydroxy3-hydroxymethyl-1-cyclopentyl)-6H-purin-6-one comprising hydrolysing a compound of formula (2) (as defined in Claim 1).
3. A process according to Claim 1 or Claims 2 wherein the hydrolysis is effected in the presence of an acid.
4. A process according to Claim 1 or Claim 2 wherein the hydrolysis is effected in the presence of hydrochloric acid.
5. A process according to Claim 4 wherein the hydrolysis is effected under reflux.
6. The use of Compound A for the manufacture of a medicament for the therapy or prophylaxis of Herpesviridae infections in a human or non-human animal subject.